# EUROPEAN PATENT APPLICATION

(11) **EP 0 846 467 A1**
(43) Date of publication of application: **10.06.1998**
(21) Application number: 97924028.0
(22) Date of filing: 29.05.1997
(51) Int. Cl.: A61K 35/78

(54) **PROCESS FOR THE PREPARATION OF AN EXTRACT USED AS A BASE MATERIAL TO OBTAIN MEDICAMENTS USEFUL FOR THE TREATMENT OF HUMAN AILMENT OF VIRAL ORIGIN**

(30) Priority: 29.05.1996 ES 9601181; 25.02.1997 ES 9700401
(71) Applicant: Prodebio, S.L., 47014 Valladolid (ES)
(72) Inventor: JIMENEZ LOPEZ, Pilar, E-47014 Valladolid (ES); GIRBES JUAN, Tomás, E-47014 Valladolid (ES)
(74) Representative: Urizar Anasagasti, José Antonio
(86) International application number: ES9700137
(87) International publication number: WO9745134

(57) **Abstract**

Process for the preparation of an extract used as a base material to obtain medicaments intended to the treatment of human ailments of viral origin, such medicinal preparation being provided in the form of, for example, syrups, pills, tablets, powder, caramels and the like, used as medicaments for the treatment of human diseases of viral origin, said preparation process comprising a phase for obtaining an homogeneous mixture of elder fruits, bark of elder branches and elder leaves, a phase of chopping the ligneous material of said mixture, a phase for adding boiling water to said mixture, a phase of decoction of the mixture, a phase for removing the solids from the compound product thus obtained, the invention relating also to the resulting extract as well as to the medicaments obtained from said extract.

## Description

The object of present invention consists of as indicated in the title, a process for the preparation of an extract used as a base to obtain medicaments for the treatment of viral origin human diseases, of the type of preparations and syrups used as medicines for the treatment of dual origin human diseases, such as influenza, colds, pharyngitis or non bacterian throat inflammation, and generally those diseases caused by viruses having the ribonucleic acid as a genetic material, the extract obtained through said process, as well as products obtained from that extract that presents outstanding therapeutic qualities.

Traditionally, plants have represented a cure for a great number of human diseases, thus leading to consider many of them as medicines, despite that in many cases we do not know the active principle and/or the process as well as the chemical base for acting.

Elder {"Sambucus nigra L."] occupies an outstanding place among a great number of medicinal plants, described in books on phytotherapy. Thus, its flowers infusion are useful for the treatment of colds, bronchial fever and hay fever {R. Mabey et al. "A new era for herbs" Ed. Everest, Leon 1988}. On the other hand, elder base ointments are useful for the treatment of chilblains and skin chafing. Decoction of elder fruits has been traditionally used for the treatment of cough and colds {P. Font Quer. Medicinal Plants. The renewed dioscorides. Ed. Labor SA, 11 eddition Barcelona 1988. R. Mabey et al "A new era for herbes" Ed. Everest, Leon 1988}.

Plants chemical mixture is very complex as they are live beings and contain secondary metabolism products. From the phytotherapy point of view, the different parts of plants, in general, and of elder in particular, present different mixtures. Thus, elder fruits are rich in C vitamin, organic acids, bioflavonoids, etc; its leaves are rich in various vitamins, tannins, resins, cianogenic type glucosides. etc, and its flowers are rich in terpenes, flavonoids, etc {R. Mabey et al "A new era for herbs" Ed. Everest, Leon 1988}

Furthermore, elder contains different functional proteins of therapeutic action, and in particular certain ribosome inactivating lectines and proteins.

In particular, lectines are fructose fixing proteins and can, therefore, interfere in identification processes of cells, viruses, bacteria, etc. Lectines known to be in elder are: SNA {"Sambucus nigra agglutinin") I and SNA II in the bark, and SNA III in the fruits. SNA I as well as SNA II stick to the intestine mucous membrane and, partially, are absorbed by the blood through said membrane. (A. Pusztai et al "Lectin Reviews", vol. 1. pg. 1-15; 1991], following a process that requires receptor mediated endocitosis {A. Pusztai et al. "Digestion", vol. 46, sup. 2, pgs. 308-316, 1990}. With respect to SNA III {now called SNA IV in the reference Van Damme et al, "Eur. J. Biochem" vol. 237, pgs. 505-513, 1996}, contained in elder fruits, we do not know of any study made about fixing and absorption to intestine mucous membrane.

Ribosome inactivator proteins are inhibitors of protein biosynthesis {Gasperi-Campani et al, "Biochem J." vol. 186, pgs. 439-441, 1980; Gasperi-Campani et al, "J. Nat. Prod.", vol. 48, pgs. 446-454, 1985; Ferreras et al, "Cell, Mol, Biol.", vol. 35, pgs. 89-95, 1989; Merino et al, "J. Exp. Bot.", vol. 41, pgs. 67-70, 1990; Girbes et al, "Cell.Mol.Biol.", vol. 38, pgs. 803-812, 1992; Citores et al, "Cell.Mol.Biol.". vol. 39, pgs. 885-995, 1993; Stirpe et al, "Bio/technol", vol. 10, pgs. 405-412, 1992; Citores et al. "FEBS Lett", vol 329, pgs. 59-62, 1993}. These proteins may be formed by one polypeptidic chain {type 1}, or by two polypeptidic chains {type 2}, or by four polypeptidic chains {type 4}. {Citores et al "FEBS Lett", vol. 329, pgs. 59-62, 1993}. Those formed by one plypeptidic chain have activity N-glucoside of ribosome nucleic acid {A chain} and one lectine {chain B} {Stirpe et al, "Bio/technol". vol. 10, pgs. 405-412, 1992}, which normally identify rests of galactose and its derivatives. Four chain proteins are made of A-B type dimers, each of them being similar to a type 2 molecule {Citores et al, "FEBS Lett, vol. 329, pgs. 59-62, 1993}. These proteins {A and B} have a molecular mass between 20.000 and 34.000. A chain block ribosomic action through inactivation catalitic process of ribonucleic acid {Jimenez y Vazquez, "Ann. Rev. Microbiol.", vol. 39, pgs. 649-672, 1985; Roberts & Selitrennikoff, "Biosc.Rep." vol. 6, pgs. 19-29, 1986; Stirpe & Barbieri,"FEBS Lett", vol. 195, pgs. 1-8, 1986; Stirpe & col. "Bio/technol", vol. 10, pgs. 405-412; Citores & col. "FEBS Lett". vol. 329, pgs. 59-62, 1993; Girbes et al, "J.Biol.Chem", vol. 268, pgs. 18195-18199, 1993; Girbes et al, "Plant.Mol.Biol.", vol-22, pgs. 1181-1186, 1993}.

Three RIP models, with two nigrine chains, have been identified in elder, and published: b nigrine in bark {Girbes et al, "Plant.Mol.Biol.", vol. 22, pgs. 1181-1186, 1993}, f nigrine in the fruits {Girbes et al, "Biochem.J.", vol. 315, pgs. 343-344, 1996; Girbes et al "J.Exp.
Bot." vol. 47, pgs. 1577-1785, 1996} and s nigrine in the seeds {Citores et al. "J.Exp.Bot.", vol 45, pgs. 513-516, 1994}. They all also show in B chain some activity of D-galactose specific lectine.

Recently, SNA lectine has been described as a real RIP {Van Damme et al, "Eur.J.Biochem", vol 235, pgs. 128-137, 1996}, what would explain the relative toxicity of that protein on intestine fixing and absorption tests. {A.Pusztai et al, "Lectin Reviews", vol. 1, pgs. 1-15, 1991}. According to recent experimental data {Batelli et al, "Arch. Toxicol", press 1996}, nigrine is a toxic substance for Swiss race rats having 30 gr. body weight {LD50-average lethal dose- to 15 days with 12 mg/kg. of body weight}.

Finally we should mention that there is a complex preoparation, which include elder fruits extracts, named Sambucol {W. Elliman, "Hadassah Magazine", pgs. 40-41, December 1994}, which contains elder and currant extracts among other components. Also, it contains non denatured native elder fruit proteins. Its active principles, as previously described, are non denatured native proteins with lectine action, contained in the preparation, and absorbed by the blood through ingestion, performing on it an antiviral action as indicated in the information available {W.Elliman, "Hadassah Magazine", pgs. 40-41, December 1984}. It is claimed that said product presents anti flu properties and, in general, antiviral properties. Since lectines and RIPs have a certain toxicity. The use of preparation that contains non denatured elder proteins implies some risks {A.Pusztai et al, "Lectin Reviews", vol. 1, pgs. 1-15, 1991}, which need to be considered before being applied on human beings. In fact, it has been known for some years that drinking raw or unadequately cooked elder fruit juices may produce nausea and vomits in a degree related to the fruit rawness {Bergman, "Flussiges Obst., vol. 46, pg. 8, 1979; Kuglmann, "Lebensm Rdsch", vol. 75, pg. 390, 1979}. It is known that fruits containing toxic bioactine proteins might be the origin of these effects. Among those proteins are f nigrine in the fruit pulp {Girbes et al "Biochem J", vol. 315, pgs. 343-344, 1996; Girbes et al "J.Exp.Bot." vol. 47, pgs. 1577-1785, 1996}. s nigrine in the seeds {Citores et al "J.Ex.Bot." vol. 45, pgs. 513-516, 1994} and SNA IV lectine in the fruit pulp {Mach et al, "Biochem J." vol. 278, pgs. 667-671, 1991}.

On the other hand, lectines as well as eldes RIPs are very amynologic {Girbes et al, "J.Exp.Bot.", vol. 47, pgs. 1577-1785, 1996}, so once ingested and absorbed by the blood plasma, they develop neutralizing antibodies, which prevent these proteins from potential action. With the purpose of taking efficient advantage of therapeutic qualities of different elder components, and with the aim to correct properly some of the deficiencies found in the preparation obtained from said plant, a process has been developed for the preparation of a base extract for the production of medicines for the treatment of viral origin human diseases; the extract obtained through said process as well as those products obtained from that extract, enjoy outstanding therapeutic properties, which are the object of present invention.

This present invention refers to the preparation process of a base extract used to obtain medicaments for the treatment of viral origin human diseases and to the product obtained through mentioned process, and consists of a complex, heat denatured, aqueous substance, developed from elder ripe fruits, bark and leaves {Sambucus nigra}, that combines, in a sinergetic way, therapeutic properties of each of them, thus preventing potential toxicity of non denatured elder proteins.

The process for the preparation of extract obtained from elder ripe fruits, bark and leaves, as a base product used to obtain medicaments for the treatment of viral origin human diseases, as the object of present invention, is based on a sucessive application of different phases of the process, in order to obtain a final product, where factors such as needed components, doses and time spent on the different phases, as well as other manufacturing factors, are of vital importance.

Furthermore, the process, as the object of present invention, includes:
A phase to obtain an homogeneous mixture of elder fruits, branches bark and leaves, with the following percentages:
- Elder fruits: 66-72 %
- Elder branches bark: 11-14 %
- Elder leaves: 17-20 %
which refer to weight percentages;
A phase for chopping up the ligneous material obtained through previous phase, in order to get pieces of not more than 1 cm.

A phase on which the homogeneous mixture obtained through previous process is added to boiling water, in a proportion of 70-90 grams and, more preferably between 75 and 85 grams of mixture per litre of water
A phase of decoction, by heating, for, preferably, 45 minutes, of the previous mixture, and stopping the process after that period of time
A phase consisting of eliminating all solids from the extract produced through previous process; this include a first decanting subphase and a second filtering subphase, preferably by means of a sieve,

Elder fruits used for the mixture preparation, during the first phase, are to be, preferably, soil ripe fruits, of dark red wine color. Elder branches used for the bark to be included in the first phase mixtures, are to be, preferably, yearly young branches. Also, elder leaves used in the mixture preparation have to be, preferably, adult or matured leaves of, preferably 5 to 12 cms. of their longest axe and preferably between 7 and 10 cms.

Also, each elder component of the product, which takes part in the process, object of present invention, may be found in fresh condition or frozen at a temperature below -15 °C, and, preferably, between -15 °C and -30 °C.

Optionally, individual decoctions of elder fruits, leaves and bark can be done with the same weight/volume proportions, and then be mixed with individual extracts, in order to obtain the complete extract.

The final extract obtained through the solids elimination phase can be processed through the different treatment phases, in order to prepare the said substance as an aqueous decoction fluid or syrup, pills or tablets, powder or liquid or solid caramel.

In order to prepare the product from the complete extract, as an aqueous substance or syrup, one part in volume of the complete extract, obtained through the solids elimination phase, must be mixed with, preferably, one part in volume of pure bees honey, to get an homogeneus product. This homogeneous mixture is poured into a container, preferably opaque or topace color, to prevent that lights alter the preparation chemical components properties.

In order to prepare the product from the complete extract, as a solid or liquid caramel, one part in volume of said extract must be mixed with, preferably, one part in volume of honey or hydrolized caramelized cereals.

Optionally, the extract obtained through the solids elimination phase, can be combined with other plants extracts and with other substances and products, to obtain a preparation which should not substantially modify the basic therapeutic properties provided to elder components. Among those plants are Filipendula ulmaria, Primula veris, Borago officinalis, Hyssopus officinalis, Echinacea angustifolia, Eupatorium perfoliatum, Eupatorium cannabium, Achillea millefolium, Mentha piperita, Mentha spicata, Eucalyptus globulus, Thymus vulgaris, Salvia officinalis, Rosmanus officinalis, Tussilago farfara, Hypericum perforatum and Melissa officinalis.

The complete extract obtained from elder, through the described process, enjoys antiviral therapeutic properties, evidenced when applied to human beings for fighting different viral origin diseases and ailments, such as influenza, colds, nose and pharynx congestion, as well as non bacterian sore throat.

Also, the use of extract as base product to obtain medicaments for the treatment of viral origin human diseases, which has been obtained through process covered by present invention, is applied to human diseases such as hepatitis A, C, D and E, pathogenesis caused by Epstein-Barr virus, and to AIDS, caused by human immunodefficiency virus of type 1 {VIH-1} or type 2 {VIH-2}.

In order to better understand the object of present invention, we are going to describe a preferential practical application of the method used to prepare a base extract to obtain medicaments for the treatment of viral origin human diseases, which is the object of present invention, as well as tests made on sick persons with results obtained, which do not try to be limited in scope.

Present practical application of the methodused for the preparation of a base extract to obtain medicaments for the treatment of viral origin human diseases, similar to the preparations and syrups used as medicins for the treatment of viral origin human diseases, like influenza, colds, nose an pharynx congestion and non bacterian throat inflammation or sore throat, and, in general, those diseases caused by viruses with ribonuecleic acid {ARN}as genetic material, starts with the selection of elder components which are to be used in the application of the said method, like fruits, branches bark and leaves.

Thus, elder fruits used in the mixture preparation are soil ripe fruits, of dark red wine color. Elder branches bark used in the mixture should, preferably, be youmg yearly branches. Also, elder leaves used in the mixture shoul, preferably, be adult or ripe leaves, being their longest axis between 7 and 10 cms.

For present practical application we mix add 55 grams of elder dark red wine color ripe fruits {fresh or frozen at -20 °C} 10 grams of yearly branches bark {raw or frozen at -20 °C} and 15 grams of yearly ripe elder leaves with their longest axis being 7 to 10 cms. {frozen at -20 °C or raw}.

Following that, mixture ligneous material shall be chopped up to obtain pieces of not more than 1 cm.

After that, this mixture shall be added to one litre of boiling water, keeping it boiling for 45 minutes.

Once this boiling process is finished, solid material will be eliminated, first through a decanting process and later by filtrating it with a sieve.

The complete extract, obtained through this process, can be subject to different presentation processes, such as aqueous decoction or syrup, pills or tablets, powder or solid or liquid caramel.

In order to prepare the product, from the final extract, as an aqueous substance or syrup, we will mix one part in volume of the complete extract, obtained through the solids elimination phase, with one part in volume of pure bees honey. As an option, we can, also, mix one part in volume of the extract concentrated solution and one part in volume of hydrolized cereals.

Therapeutical efficiency of the complete extract obtained from elder, through the process described, enjoys antiviral therapeutical properties, evidenced when sucessfully fighting severeal viral origin human diseases, such as influenza, colds, nose and pharynx congestion as well as non bacterian sore throat as well as other applications previously mentioned and based on the results obtained through a series of tests made on patients affected by mentioned diseases, which are described hereafter. In those tests, in the cases of influenza and colds, the etiologic agents are ARN {ribonucleic acid} type viruses; influenza virus types A, B and C {D.P. Stites & AL Terr, "Basic & Clinical Inmunology", pgs. 646-656, Lange Medical Book, Appleton & Lange, Norwalk, Conneticut, USA, 1987} and rhinovirus in the case of colds
{D.J. Jeffries& E. De Clerk, "Antiviral chemotherapy", pgs. 321-355, Jhon Wiley & Sons, New York, USA, 1995}.Test applied were the following:

### Test 1 - Application of syrup extract to patient N° 1

The extract was ingested by a 44 y.o. man of strong completion, no having chronic diseases, suffering influenza in its initial phase {first 24-48 hrs} featured by headache, nasal congestion and fever. He was given a spoonful of syrup {9 ml.} every 6 hrs. After 24 hours of treatment, influenza symptoms improved substantially, and they disappeared 24 hours later {full recovery}.

### Test 2 - Application of syrup extract to patient N° 2

The extract was ingested by a 31 y.o. woman, no having chronic diseases, suffering influenza in its initial phase {first 24-48 hrs.}, featured by headache , nose congestion fever and strong sore throat with pain when eating.. She was given a spoonful of syrup (9 ml) every 6 hours. After 24 hours of treatment, the flu symptoms improved substantially and disappeared completely 24 hours later (full recovery).

### Test 3 - Application of syrup extract to patient N°3

The extract was ingested by a 44 y.o. man, of strong completion, with no chronic diseases, suffering influenza in its initial phase (first 24-48 hours), featured by headacge and fever. He was given a spoonful of syrup (9 ml.) every 6 hours. After 24 hours of treatment symptoms desappeared (full recovery).

### Test 4 - Application of syrup extract to patient N° 4

The extract was ingested by a 9 y.o. girl, of normal development, with no chronic diseases, suffering a stron nose congestion. cold throat pain in its initial phase (first 24 hrs.). She was given a small spoonful of syrup (2 ml) every 6 hours. After 24 hours of treatment the symptoms improved substantiallyand disappeared completely after 48 hours (full recovery).

### Test 5 - Application of syrup to patient N° 5

The extract was ingested by a 43 y.o. man, of strong completion, with no chronic diseases, suffering a strong cold, nose congestion and strong sore throat. Three days later he was given a spoonful of syrup (9 ml) every 6 hours. After 24 hours of treatment the symptoms disappeared completely (full ecovery).

### Test 6 - Application of syrup on patient N° 6

The extract was ingested by a 36 y.o. man, of strong completion, with no chronic diseases, suffering a strong cold with nose congestion and a strong sore throat. Three days later he was given a small spoonful of syrup (2 ml) every 8 hours. After 24 hours of treatment his sore throat improved substantially, but the cold did not disappear (partial recovery). Treatment lasted two days. Lack of improvement in cold symptoms could be due to insufficient syrup doses.

### Test 7 - Application of syrup extract to patient N° 7

The extract was applied to a 38 y.o. man, of strong completion, with no chronic diseases, suffering influenza in its initial phase (first 24-48 hours), featured by headache, nose congestion and some fever and sore throat. He was given a spoonful of syrup (2 ml) every 6 hours. After 24 hours of treatment the flu symptoms improved substantially and disappeared completely 24 hours later (full recovery).

### Test 8 - Application of syrup extract to patient N° 8

The extract was ingested by a 41 y.o. woman, with no chronic diseases, suffering a sore throat and with symptoms of influenza in its initial phase (first 24 hours). She was given a spoonful of syrup (9 ml) every 6 hours. After 24 hours of treatment the influenza symptoms improved substantially and disappeared completely 24 hours later (full recovery).

### Test 9 - Application of syrup extract to patient N° 9

The extract was ingested by a 24 y.o. man, of medium completion, with no chronic diseases, suffering a strong sore throat. He was given a small spoonful of syrup (2 ml) every 6 hours. After 24-48 hours of treatment the symptoms disappeared completely (full recovery).

### Test 10 - Application of syrup extract to patient N° 10

The extract was ingested by a 33 y.o. man, of medium completion, with no chronic diseases, suffering influenza in its initial phase (first 24-48 hours), featured by headache, nose congestion and sore throat. He was given a small spoonful of syrup (2 ml) every 6 hours. After 24 hours of treatment the influenza symptoms improved substantially and disappeared completely 24 hours later (full recovery).

### Test 11 - Application of syrup extract to patient N° 11

The extract was ingested by a 42 y.o. woman, of strong completion, with no chronic diseases, suffering influenza in its initial phase (first 24-48 hours), featured by headache, nose congestion and sore throat. She was given a small spoonful of syrup (2 ml) every 6 hours. After n24 hours of treatment, the influenza symptoms improved substantially and disappeared completely 24 hours later (full recovery). Bronchitis broke out and did not disappear during treatment period beacause of previous condition.

### Test 12 - Application of extract syrup to patient N° 12

The extract was ingested by a 8 y.o. child, of normal development, with no chronic diseases suffering pain and a strong cold with throat pain in its initial phase (first 24 hours). He was given a small spoonful of syrup (2 ml) every 6 hours. After 24 hours of treatment, the symptoms improved completely (full recovery).

### Test 13 - Application of extract syrup to patient N° 13

The extract was ingested by a 43 y.o. man, of medium completion, with no chronic diseases but with stomach problems, suffering a cold and nose and pharynx congestion in its initial phase (first 24 hours) with 37 °C fever. He was given a spoonful of syrup (9 ml) every 6 hours). After 24 hours of treatment fever disapppeared and the patient improved substantially. Treatment lasted 1,5 days, and might have been too short. Treatment was reinitiated three days later and lasted 24 hours, and symptoms disappeared completely
(full recovery).

### Test 14 - Application of extract syrup to patient N° 14

The syrup was ingested by a 7 y.o. child, of normal development, with no chronic diseases, suffering a cold nose and pharinx congestion in its initial phase (first 24 hours), with 37 °C fever. He was given a small spoonful of syrup (2 ml) every 6 hours (total amount 6 ml). After 24 hours of treatment, fever and symptoms disappeared completely (full recovery).

### Test 15 - Application of extract syrup to patient N° 15

The syrup was ingested by a 8,5 y.o. child, of normal development, with no chronic diseases, suffering a cold with strong coughing and plenty of mucosity in its initial phase (first 24 hours). He was given a small spoonful of syrup (2 ml) every 6 hours (total amount 6 ml). After 24 hours of treatment symptoms disappeared completely (full recovery).

### Test 16 - Application of extract syrup to patient N° 16

The syrup was ingested by a 45 y.o. man, of strong completion, with no chronic diseases, sufferin a strong influenza in its initial phase (first 24-48 hours), featured by a strong headache, nose congestion and fever, as well as by a strong sore throat and bronchitis. He was given a spoonful of syrup (9 ml) every 6 hours. After 48 hours of treatment the influenza symptoms disappeared and after 72 hours the sore throat also disappeared. Treatment lasted only three days. Bronchitis disappeared the fourth day from start of treatment.

### Test 17 - Application of extract syrup to patient N° 17

The syrup was ingested by a 7,5 y.o. child, of normal development, with no chronic diseases, suffering congestion, throat pain (strong chafing) and light bronchitis in its initial phase (first 24 hours). She was given a small spoonful of syrup (2 ml) every 8 hours. After 48 hours of treatment the throat pain and congestion disappeared but bronchitis remained for three more days. Treatment lasted two days..

### Test 18 - Application of extract syrup to patient N° 18

The syrup was ingested by a 12,5 y.o. girl, of normal development, with no chronic diseases, suffering a cold process. She was given a spoonful of syrup (2 ml) every 6 hours. After 48 hours of treatment the cold process disappeared. Treatment lasted two days.

### Test 19 - Application of extract syrup to patient N° 19

The syrup was ingested by a 45 y.o. man of strong completion, with no chronic diseases, suffering strong influenza in its initial phase (first 24 hours), with an intense throat pain. He was given a spoonful of syrup (9 ml) every 6 hours. After 48 hours of treatment, that condition disappeared. The sore throat disappeared in four days. The treatment lasted two days.

### Test 20 - Applicationof extract syrup to patient N° 20

The syrup was ingested by a 50 y.o. woman suffering influenza in its initial phase (first 48 hours), strong coughing, congestion, featured by headache and fever. She was given a spoonful of syrup (9 ml) every 6 hours. After 24 hours of treatment the symptoms disappeared (full recovery).

### Test 21 - Application of extract syrup to patient N° 21

The syrup was ingested by 26 y.o. man, of normal physical development, with no chronic diseases, except for stomach affection due to an ulcerous process, and suffering influenza in its initial phase, congestion, moderate fever and headachein their initial phase (first 24 hours). He was given a spoonful of syrup (9 ml) every 6 hours. After 24 hours of treatment, all symptoms practically disappeared. Treatment consisted of three doses. Light stomach pain was detected inmediately after stopping treatment.

### Test 22 - Application of extract syrup to patient N° 22

The syrup was ingested by a 41 y.o. man, with no chronic diseases, suffering a very strong influenza condition and a nose cold. He was given a spoonful of syrup (9 ml) every 4 hours. After 48 hours of treatment, the influenza symptoms disappeared but cold symptoms did not. Treatment started three days after symptoms appeared and lasted 4 days. The cold persisted after the treatment.

Once the characteristics of present invention as well as the method to put it into practice have been described sufficiently, we only have to add that it will be possible to make changes in the shape, materials and arrangement, as long as those changes will not modify substantially the invention characteristics which are claimed as follows:

## Claims

1. Method used for the preparation of a base extractintended to obtain medicaments for the treatment of viralorigin human diseases, like those preparations syrups, pills, tablets, powder, caramels and the like, which will be used as medicines for the treatment of human diseases of viral origin, such as influenza, colds, nose and pharynx congestion and non bacterian throat inflammation or sore throat, as well as, in general, those diseases caused by viruses with ribonucleic acid (ARN) as genetic material, featured by having the following phases:
Phase for obtaining an homogeneous mixture of elder fruits, branches bark and leaves, with following quantitative percentages
Elder fruits 66-72 %
Elder branch bark 11-14 %
Elder leaves 17-20 %
which refer to weight percentages.
Phase for chopping up the ligneous material of the homogeneous mixture obtained through previous phase, to obtain pieces of not more than 1 cm.
Phase for the addition of the homogeneous mixture, obtained through previos phases, to boiling water, in a proportion of 70-90 grams, or preferably between 75 and 85 grams of mixture per liter of water.
Phase of decoction, consisting of boiling during, preferably, 45 minutes the mixture obtained through previous phases, finishing this heating process after that time.
Phase for the elimination of solids from the extract obtained through previous phase, consisting of, first a decating process, and, second, a filtering process, using a sieve.

2. Method for the preparation of a base extract used to obtain medicaments for the treatment of human diseases of viral origin, in accordance with the mentioned claim (par. 1), featured by the fact that elder fruits used to obtain the homogeneous mixture, or first phase, are preferably to be soft ripe fruits of dark red wine color, and that elder branches bark used to obtain the homogeneous mixture, or first phase, are to be, preferably, young branches, of less than one year, and that elder leaves used to obtain the homogeneous mixture, in the first phase, are to be, preferably, adult or ripe leaves, with, preferably, their longest axis of 5-12 cms., and preferably between 7 and 10 cms.

3. Method for the preparation of a base extract used to obtain medicaments for the treatment of human diseases of viral oirigin, in accordance with mentioned claims, and featured by the fact that each of the elder components which take part in the said methodcan be found in raw condition or frozen, at a temperatura below -15 °C, and preferably between -15 °C and -30 °C.

4. Method for the preparation of a base extract used to obtain medicaments for the treatment of human diseases of viral origin, in accordance with mentioned claims, and featured by the fact that individual decoctions with elder fruits, leaves and bark can be prepared with the same weight/volume proportions, and then be combined with the individual extracts to obtain the complete extract.

5. Extract obtained by method in accordance with claims 1 to 4.

6. Extract refered to in claim 5, featured by the fact that said extract can be subject to different treatment processses to obtain medicaments as all aqueous solution or syrup, pills or tablets, powder or liquid or solid caramel.

7. Medicament obtained from mentioned extract, refered to in claim 5, and featured in that it consists in an homogeneous solution of one part in volume of said extract and one part in volume of pure bees honey.

8. Medicament obtained from the extract refered to in claim 5, featured in that it consists, preferably, of a homogeneous solution of one part in volume of a concentrated solution of said extract and one part in voluime of hydrolized cereals.

9. Medicament obtained from extract refered to in claim 5, featured in that it is held in an opaque or topace color container.

10. Medicament obtained from the extract refered to in claim 5, and featured in that it consists of a caramelized mixture of one part in volume of said extract and, preferably, one part in volume of honey or caramelized hydrolized cereals.

11. Medicament obtained from the extract refered to in claim 5, and featured in that it consists of a combination of said extract with the extract of, at least, another plant, being this plant taken preferably from the following: Filipendula ulmaria, Primula veris, Borago officinalis, Hyssopus officinalis, Echinacea angustifolia, Euypatorium perfoliatum, Eupatorium cannabium, Achillea millefolium, Mentha piperita, Mentha spicata, Eucalyptus globulus, Thymus vulgaris, Salvia officinalis, Rosmarinus officinalis, Tussilago farfara, Hypercium perforatum, and Melissa officinalis.

12. Application of medicament obtained from the extract obtained by the method refered to in claim 1, and featured by the fact that it also will be applied to treatment of hepatitis A, C, D and E.

13. Application of medicament obtained fron exttract obtained by the method refered to in claim 1, and featured by the fact that it also will be applied to treatment of human inmunodefficiency syndrome (AIDS), caused by human inmunodefficiency viruses of type 1 (VIH-1) or type 2 (VIH-2).

14. Application of medicament obtained from extract obtained by method refered to in claim 1, and featured by the fact that it will also be applied to the pathogenesis caused by the Epstein-Barr virus.
